# EUROPEAN PATENT APPLICATION

(11) **EP 3 967 330 A1**
(43) Date of publication of application: **16.03.2022**
(21) Application number: 20306002.5
(22) Date of filing: 10.09.2020
(51) Int. Cl.: A61K 49/00, A61P 35/00

(54) **METHODS FOR THE SYNTHESIS OF BIOACTIVATED METAL NANOCLUSTER AND THEIR MEDICAL APPLICATION**

(71) Applicant: Université de Rennes 1, 35000 Rennes (FR); CENTRE NATIONAL DE LA RECHERCHE SCIENTIFIQUE -CNRS-, 75016 Paris (FR); Institut National des Sciences Appliquées de Rennes, 35708 Rennes Cedex 7 (FR); Ecole Nationale Superieure de Chimie de Rennes, 35700 Rennes Cedex (FR)
(72) Inventor: MARCHI, Valérie, 35000 Rennes (FR); CHIECHIO, Regina Maria, 95032 Belpasso (IT); EVEN-HERNANDEZ, Pascale, 35760 Saint-Gregoire (FR)
(74) Representative: Plasseraud IP

(57) **Abstract**

The present invention relates to a bioactivated metal nanocluster comprising a mixture of ligands linked to a fluorescent metallic nanocluster, their synthesis methods, and their medical applications.

## Description

### TECHNICAL FIELD

The present disclosure relates to methods for the synthesis of bioactivated metal nanoclusters, and their medical application.

### BACKGROUND

Cancer remains one of the most devastating diseases in the world with more than 10 million new cases every year. The rate of growth of tumour masses is impressive, as tumour cells replicate much faster than healthy cells; hence the need for treatments that are sufficiently effective to counteract the rate of tumour cell development while minimizing the destruction of healthy tissues. The treatments currently used to treat cancer are generally intrusive processes with many side effects: chemotherapy to shrink the tumour, followed by surgery to remove it when possible, and additional chemotherapy and radiotherapy.

Over the past 25 years, much research has increased the effectiveness of therapies, but these improvements are not yet sufficient. Current studies focus on creating vectors to deliver the drug directly to the site of interest (drug delivery), finding new targets to attack (e.g. blood vessels that feed tumour growth) and try to improve current treatments by making them more effective and selective. The objective of these studies is to increase the quality of life and life expectancy of patients.

One of the conventional methods used to treat cancer is chemotherapy. Conventional chemotherapeutic agents are distributed non-specifically in the body and therefore damage both cancer and healthy cells: this limits the dose received by the cancer cells and makes the treatment excessively toxic to the body.

The other widely used method of cancer treatment is radiotherapy, which is used in about 50% of patients. Similar to chemotherapy, one of the main problems with this technique is the non-specificity, as the beams are able to kill both cancerous and healthy cells. It is therefore extremely important, using an improved imaging technique, to precisely define the volume of the tumour mass and to adjust the radiation dose administered to the patient. A state-of-the-art technique is the use of radiosensitizers that are capable of increase the effectiveness of radiation therapy by increasing the damage to the cell. The most common radiosensitizers are chemotherapeutic types and are intended to reduce the resistance of tumour cells, for example by preventing the formation of the blood capillaries that nourish them.

In recent years, the possibility of using nanoparticles (NPs) to improve existing therapies has been explored. The ability to select the surface ligands of such particles makes it possible to use recognition ligands that preferentially bind to tumor cells while adding fluorophores for imaging and diagnosis. In application to drug delivery, this method allows the transport of chemopharmaceuticals or radiopharmaceuticals to the NP surface and their preferential release directly to the tumor area. Thanks to nanoparticles, it is therefore possible to increase the specificity of treatments and to allow the drug to enter directly into the cell to increase its effectiveness. NPs, by binding to the cell via specific receptors, are able to bypass the recognition of P-glycoprotein (one of the main defence mechanisms) and penetrate the cell's immune system usually in the cell by endocytosis.

NPs have also played the innovative role of radio sensitizers in recent years, although it's still only at the experimental research level. The operating principle is based on the ability of the metal to absorb, scatter and re-emit incident radiation by X-rays, photoelectrons, Compton electrons and Auger electrons causing radiochemical damage (free radicals and ionization). Thanks to the recognition ligands, the NPs are mainly located on the surface or inside the tumor cells, thus allowing the exaltation of radiation preferentially at the tumor level. This is due to the fact that elements with a high atomic number (Z) have a higher energy absorption coefficient than soft tissue.

Among these emerging radiosensitizers, gold nanoparticles (AuNPs) are particularly attractive due to their strong interaction with radiation (Z_{Au} = 79), good biocompatibility and excellent chemical stability. However, most AuNPs are generally larger than 50 nm, which induces their capture and absorption by the reticuloendothelial system (RES) and thus leads to their accumulation in the liver and spleen. Reducing the size of AuNPs (<20 nm) would avoid this problem as the particles could easily escape the reticuloendothelial system (RES). However, to be expelled from the renal system, the size must be even smaller (<5.5 nm). If the particles cannot be eliminated by the kidneys, they accumulate in the body, making them toxic in the long term.

The technical solution to this problem could therefore be the use of AuNPs with sizes below 2 nm, which are called nanoclusters (NCs). In combination with ligands such as glutathione (GSH), it guarantees excellent biocompatibility and small hydrodynamic diameter (HD). In addition, it has also been shown that by decreasing the size, the efficiency of radiosensitization increases. However, and until now, there is no ligand capable of being at the same time highly fluorescent, small (<5.5 nm), photostable and at the same time recognizing a predefined target.

We sought to develop a single step synthesis of bioactivated metal nanoclusters comprising ligands that specifically recognize a predefined target, and preferably, receptors expressed by cancer cells.

### SUMMARY

The present inventors have now found a way to design and produce a bioactivated metal nanocluster which is chemically stable and photostable even if stored at ambient or short term elevated temperatures so that it can be produced on commercial scale and supplied either as ready-to-use pharmaceutical product or as an active ingredient in a specific drug formulation.

One first aspect of the invention relates to a bioactivated metal nanocluster comprising a mixture of ligands linked to a fluorescent metallic nanocluster, wherein the mixture comprises at least ligands linked to a fluorescent metallic nanocluster of formula (Ib) :

Y-AₚE_{n'}(X₂) _{(Ib)}

wherein
Y, being on the N terminal side of A, is the fluorescent metallic nanocluster wherein the metal is selected from the group consisting of gold, copper, silver, platinum, and nickel;
AₚE_{n'}(X₂), is a matrix ligand of formula (Ib);
A is an anchor composed of an (L) or (D) amino acid selected from the group comprising Glycine (Gly), Alanine (Ala), Valine (Val), Cysteine (Cys), Proline (Pro), Leucine (Leu), Isoleucine (Ile), Methionine (Met), Tryptophan (Trp), Phenylalanine (Phe), Lysine (Lys), Arginine (Arg), Histidine (His), Threonine (Thr), Tyrosine (Tyr), Serine (Ser), Asparagin (Asn), Glutamine (Gln), Aspartic Acid (Asp), Glutamic acid (Glu), and mixture thereof;
p is an integer greater than 1, preferably between 1 to 10, more preferably between 2 and 6, even more preferably 3 ;
E, being on the C terminal side of A, is a spacer composed of ethylene glycol unit repeated n' times;
n' is an integer greater than or equal to 1, preferably between 2 to 20, more preferably between 3 and 8, even more preferably 4 or 6 ;and
X₂ is a matrix ligand selected from the group comprising at least one (L) or (D) amino acid or a primary amine (-RNH₂) or alcohol (-RCH₂OH) or alkyl carboxylic acid (-RCO₂H) or alkyl ether group (-ROR'), wherein the matrix ligand does not contain thiol function and R and R' are short alkyl chain.

In another aspect, the invention relates to a pharmaceutical composition comprising:
a. The bioactivated metal nanocluster of the invention, and
b. At least one pharmaceutical acceptable excipient.

It is another object of the present invention to provide a method for the synthesis of the bioactivated metal nanocluster of the invention, wherein said method comprises the following steps :
a. Providing, an aqueous solution comprising at least the ligands of formula (Ib) as defined in the invention and water,
b. Adding a solution of the metal salts selected from the group consisting of gold, copper, silver, platinum, and nickel, preferably a solution of gold salts of formula HAuCl₄,
c. Reacting during sufficient period of time at a temperature between 20°C and 200°C, preferably 70°C to 150°C, more preferably about 120°C,
d. Recovering the bioactivated metal nanocluster formed, and
e. Optionally, purifying the bioactivated metal nanocluster formed.

The present invention further relates to a bioactivated metal nanocluster of the invention obtainable by the method of the invention, wherein it has a stability at 4°C of at least 1 month, preferably at least 6 months, more preferably at least 12 months, even more preferably between 1 to 24 months, even more preferably between 4 to 18 months, even more preferably between 6 to 12 months.

Another aspect of the invention relates to a bioactivated metal nanocluster of the invention or a composition of the invention for use as a medicament.

In another aspect, the invention relates to a bioactivated metal nanocluster of the invention or a composition of the invention for use in treating cancer.

Finally, the present invention further relates to a bioactivated metal nanocluster of the invention or a composition of the invention for use in detecting cancer.

### DETAILED DESCRIPTION OF THE INVENTION

### Definitions

Terms as used throughout the invention are hereafter defined.

As used herein, the term "cancer" refers to cells having the capacity for autonomous growth which is an abnormal state or condition characterized by rapidly proliferating cell growth. The term is meant to include all types of cancerous growths or oncogenic processes, metastatic tissues or malignantly transformed cells, tissues, or organs, irrespective of histopathologic type or stage of invasiveness.

The phrase "treatment of' and "treating" includes the amelioration or cessation of a disease, disorder, or a symptom thereof. In particular, with reference to the treatment of a tumor, the term "treatment" may refer to the inhibition of the growth of the tumor, or the reduction of the size of the tumor.

As used herein, the term "bioactive ligand" means ligand that specifically recognizes a predefined target, and preferably, receptors expressed by cancer cells. The bioactive ligand can have the formula Ia: ApEn(X1) or Ic: ApEn"(X₃) or mixtures thereof.

As used herein, the term "matrix ligand" means ligand which does not recognize a predefined target but is only here to dilute the surface proportion of the bioactive ligand and preserve the colloidal stability as well as the photostability of the metal nanocluster. The matrix ligand is of formula Ib: ApEn'(X₂).

As used herein, the term "metal nanocluster (NCs)" means small aggregates of metal atoms that represent an intermediate state between a molecule and a nanoparticle. They are smaller than 5,5 nm, preferably smaller than 2nm.

As used herein, the term "bioactivated metal nanocluster" means that the metal nanocluster has been linked to ligands or mixtures thereof which specifically recognize a predefined target, and preferably, receptors expressed by cancer cells.

As used herein, the term "mixture of ligands linked to a fluorescent metallic nanocluster" means that at the surface of the metal nanocluster, each metal atom is linked to a ligand. The ligand can be either a bioactive ligand or either a matrix ligand.

As used herein, the term "linked" in ligands linked to a fluorescent metallic nanocluster means that ligands are directly linked to the fluorescent metallic nanocluster. The linking bond is either covalent or non-covalent bond between the ligands and the fluorescent metallic nanocluster, preferably the bond is covalent bond and more preferably a organometallic bond.

As used herein, the term "pharmaceutical acceptable excipient" means excipient conventionally used in the pharmaceutical field which does not, and is limited only by physico-chemical considerations, such as solubility and lack of reactivity with the active compound(s).

As used herein, the terms "effective amount" or "therapeutically efficient amount" of a compound refer to an amount of the compound that will elicit the biological or medical response of a subject, preferably ameliorate the symptoms, alleviate conditions, slow or delay disease progression, or prevent a disease.

As used herein, the term "in dried form" refers to a pharmaceutical composition that has been dried , for example via spray-drying, to a powder having a moisture content below about 10% by weight, usually below about 5% by weight, and preferably below about 3%. The NCs is preferably lyophilized.

Unless otherwise defined, "%" has herein the meaning of molar percentage which is the molecule proportion in percent (%mol).

If not stated herein otherwise, "about" means ±15 minutes when it is expressed in minute, or ±5°C when it is expressed in degree, or ± 20%, preferably ± 10%, more preferably ± 5% when it is expressed in %.

As used herein, the term "short alkyl chain" refers to an alkyl chain between 1 and 3 carbon atoms, preferably 1 or 2 carbon atoms, more preferably 1 carbon atom.

### The bioactivated metal nanocluster

Nanoclusters (NCs) are made up of an aggregate of atoms smaller than 2 nm in size. The very small dimensions (comparable to the wavelength of electrons at the Fermi level) give rise to a quantum confinement effect that generates electronic levels with discretized energy and new optical properties. NCs, after quantum confinement, do not have the collective plasmon excitation typical of nanoparticles and have composition- and size-dependent fluorescence properties.

The bioactivated metal nanocluster of the invention is composed of a core which is the metal nanocluster. The surface metal atoms in the nanocluster are linked to a ligand. The ligand can be either a matrix ligand or either a bioactive ligand. Therefore, the surface of the bioactivated metal nanocluster comprises either matrix ligands, either bioactive ligands or mixtures thereof. For example, when the surface of the bioactivated metal nanocluster consists of matrix ligands, it means that the proportion of the matrix ligand is 100%.

Advantageously, the bioactivated metal nanocluster comprises a mixture of ligands linked to a fluorescent metallic nanocluster, wherein the mixture comprises at least ligands linked to a fluorescent metallic nanocluster of formula (Ib):

Y-AₚE_{n'}(X₂) _{(Ib)}

Y, being on the N terminal side of A, is the fluorescent metallic nanocluster wherein the metal is selected from the group consisting of gold, copper, silver, platinum, and nickel. Preferably Y is a fluorescent gold nanocluster of formula Au. AuNCs have a fluorescence that can vary from the visible (VIS) to the near infrared (NIR) range by simply changing the number of gold atoms.

ApEn'(X₂), is a matrix ligand of formula (Ib). The matrix ligand must possess properties adapted to the fluorescent metallic nanocluster. When the metal is gold of formula Au, A must have an affinity for gold and must be capable of reducing gold from Au(III) to Au(I) and Au(0).

A is an anchor composed of an (L) or (D) amino acid selected from the group comprising Glycine (Gly), Alanine (Ala), Valine (Val), Cysteine (Cys), Proline (Pro), Leucine (Leu), Isoleucine (Ile), Methionine (Met), Tryptophan (Trp), Phenylalanine (Phe), Lysine (Lys), Arginine (Arg), Histidine (His), Threonine (Thr), Tyrosine (Tyr), Serine (Ser), Asparagin (Asn), Glutamine (Gln), Aspartic Acid (Asp), Glutamic acid (Glu), and mixture thereof. Preferably, A is an (L) or (D) amino acid comprising a thiol function, more preferably A is an (L) or (D) Methionine (Met, M) or an (L) or (D) Cysteine (Cys, C) or an (L) or (D) Histidine (His, H), even more preferably A is an (L) or (D) Cysteine (Cys, C) and particularly A is an (L) Cysteine (Cys, C).

p is an integer greater than 1, preferably between 1 to 10, more preferably between 2 and 6, even more preferably 3.

E is a spacer composed of ethylene glycol unit. E is on the C terminal side of the anchor, A, and serves as a flexible hydrophilic spacer capable of protecting the fluorescence of the metal NC, preferably the gold NC. Advantageously, E, being on the C terminal side of A, is a spacer composed of ethylene glycol unit repeated n' time. n' is an integer greater than or equal to 1, preferably between 2 to 20, more preferably between 3 and 8, even more preferably 4 or 6. The presence of E improves colloidal stability and preserves the optical properties of the NCs.

X₂ is a matrix ligand selected from the group comprising at least one (L) or (D) amino acid or a primary amine (-RNH₂) or alcohol (-RCH₂OH) or alkyl carboxylic acid (-RCO₂H) or alkyl ether group (-ROR'), wherein the matrix ligand does not contain thiol function and R and R' are short alkyl chain.

In an embodiment, X₂ is a matrix ligand selected from the group comprising a primary amine (-RNH₂) or alcohol (-RCH₂OH) or alkyl carboxylic acid (-RCO₂H) or alkyl ether group (-ROR') wherein R and R' are short alkyl chain of between 1 and 3 carbon atoms, more preferably 1 or 2 carbon atoms, even more preferably 1 carbon atom.

In another embodiment, X₂ is at least one (L) or (D) amino acid selected from the group comprising Glycine (Gly), Alanine (Ala), Valine (Val), Cysteine (Cys), Proline (Pro), Leucine (Leu), Isoleucine (Ile), Methionine (Met), Tryptophan (Trp), Phenylalanine (Phe), Lysine (Lys), Arginine (Arg), Histidine (His), Threonine (Thr), Tyrosine (Tyr), Serine (Ser), Asparagin (Asn), Glutamine (Gln), Aspartic Acid (Asp), Glutamic acid (Glu), and mixture thereof. More preferably X₂ is one (L) or (D) amino acid selected from the group comprising Glycine (Gly), Alanine (Ala), Valine (Val), Cysteine (Cys), Proline (Pro), Leucine (Leu), Isoleucine (Ile), Methionine (Met), Tryptophan (Trp), Phenylalanine (Phe), Lysine (Lys), Arginine (Arg), Histidine (His), Threonine (Thr), Tyrosine (Tyr), Serine (Ser), Asparagin (Asn), Glutamine (Gln), Aspartic Acid (Asp), Glutamic acid (Glu). Even more preferably X₂ is an (L) or (D) Aspartic Acid (Asp, D) or an (L) or (D) Lysine (Lys, K). Particularly X₂ is an (L) or (D) Aspartic Acid (Asp, D). More particularly X₂ is an (L) Aspartic Acid (Asp, D). The presence of X₂ allows obtaining a matrix activity i.e. inert activity that does not allow the ligand to recognize any predefined target while not interacting with gold.

The bond between Y and A is either covalent or non-covalent, preferably the bond is covalent bond and more preferably an organometallic bond. The bonds between A and E, and E and X₂ are amide bonds.

According to further embodiment the bioactivated metal nanocluster further comprises ligands and mixtures thereof which are bioactive ligands. The chemical structure of bioactive ligands influences the synthesis, the physico-chemical properties and the possible catalytic applications of such bioactivated metal NCs. During synthesis, the type of bioactive ligand influences the speed of the formation process and the size of the bioactivated metal NCs thus obtained. The bioactive ligands can have the formula (Ia): ApEn(X₁) or (Ic): ApEn"(X₃) or mixtures thereof. Particularly, the mixture of ligands linked to a fluorescent metallic nanocluster can be a mixture of formula (Ia) and (Ib) or a mixture of formula (Ia), (Ib) and (Ic) or a mixture of formula (Ia) and (Ic).

Advantageously, the bioactivated metal nanocluster further comprises bioactive ligands of formula (Ia) or (Ic) or mixtures thereof:

Y-AₚEₙ(X₁) (Ia)

Y-AₚE_{n"}(X₃) (Ic)

Y, Ap, E are as defined above.

ApEn(X1), is a bioactive ligand of formula (Ib).

n is an integer greater than or equal to 1, preferably between 2 to 20, more preferably between 3 and 8, even more preferably 4 or 6.

X₁ is a marker which is either a ligand interacting or interfering with ion channels selected from calcium channels, potassium channels, sodium channels, chlorine channels, either a receptor ligand expressed by cancer cells or exosomes selected from the group comprising at least one (L) or (D) amino acid, preferably U11 polypeptide, glycine-aspartate-arginine (RGD) or any sequence comprising RGD, or microRNAs identified as cancer markers (Valadi, H.; Ekström, K.; Bossios, A.; Sjöstrand, M.; Lee, J. J.; Lotvall, J. O. Exosome-Mediated Transfer of MRNAs and MicroRNAs Is a Novel Mechanism of Genetic Exchange between Cells. Nature Cell Biology 2007, 9 (6), 654-659.), or antibodies addressed against the tetraspanin family proteins such as CD63, CD81 and CD9 (Mathivanan, S.; Simpson, R. J. ExoCarta: A Compendium of Exosomal Proteins and RNA. PROTEOMICS 2009, 9 (21), 4997-5000; Bobrie, A.; Krumeich, S.; Reyal, F.; Recchi, C.; Moita, L. F.; Seabra, M. C.; Ostrowski, M.; Thery, C. Rab27a Supports Exosome-Dependent and -Independent Mechanisms That Modify the Tumor Microenvironment and Can Promote Tumor Progression. Cancer Research 2012, 72 (19), 4920-4930;Yoshioka, Y.; Konishi, Y.; Kosaka, N.; Katsuda, T.; Kato, T.; Ochiya, T. Comparative Marker Analysis of Extracellular Vesicles in Different Human Cancer Types. Journal of Extracellular Vesicles 2013, 2 (1), 20424), wherein the marker does not contain thiol function. The presence of X₁ allows obtaining a bioactivity, i.e. to specifically recognize a predefined target, and preferably, ion channels or receptors expressed by cancer cells or exosomes, while not interacting with gold.

In an embodiment X₁ is a ligand interacting or interfering with the calcium channel such as a calcium channel blocker selected from the group consisting of dihydropyridine such as Amlodipine, Aranidipine, Azelnidipine, Barnidipine, Benidipine, Cilnidipine, Clevidipine, Efonidipine, Felodipine, Isradipine, Lacidipine, Lercanidipine, Manidipine, Nicardipine, Nifedipine, Nilvadipine, Nimodipine, Nisoldipine, Nitrendipine, and Pranidipine, or non-dihydropyridine such as Verapamil, Fendiline, Gallopamil, Diltiazem, gabapentin and pregabalin.

In another embodiment, X₁ is a ligand interacting or interfering with the calcium channel ORAI1, such as the compound 1-[4-(4-Methoxyphenethoxy)phenethyl]-1H-benzo[d]imidazole of formula A:

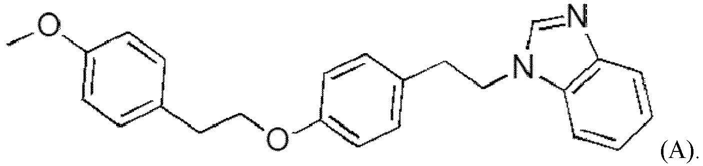

In this embodiment, in order to graft the compound of formula A onto the spacer E, which is a spacer composed of ethylene glycol unit repeated n' times, the compound of formula A must be substituted, preferably by nucleophile substitution, at one of its ends by an amino group such as a primary amine (-RNH₂). Preferably, a primary amine is substituted at the end wherein the ether group is present.

When X₁ is a ligand interacting or interfering with the calcium channel it allows to determine the level of underexpression or overexpression of these calcium channels. The underexpression or overexpression being a warning sign. For example, the overexpression of the calcium channels ORAI1 may indicate that the patient has cancer. The level of overexpression can then be quantified to help diagnose cancer. Preferably, the underexpression or overexpression of these calcium channels ORAI1 may help diagnose pancreatic and/or testicular cancers.

The bond between Y and A is either covalent or non-covalent, preferably the bond is covalent bond and more preferably an organometallic bond. The bonds between A and E, and E and X₁ are amide bond.

ApEn"(X₃), is a bioactive ligand of formula (Ic).

n" is an integer greater than or equal to 1, preferably between 2 to 20, more preferably between 3 and 8, even more preferably 4 or 6.

X₃ is a medicine selected from the group comprising anticancer medicines, an oligonucleotide selected from the group comprising anticancer medicines, or microRNAs (miRNAs) coding proteins of therapeutic interest, wherein the medicine or the oligonucleotide does not contain thiol function. The presence of X₃ allows obtaining a bioactivity i.e. to specifically recognize a predefined target, and preferably, ion channels or receptors expressed by cancer cells, while not interacting with gold.

In an embodiment X₃ is an anticancer medicine selected from the group consisting of docetaxel, paclitaxel, methotrexate, pemetrexed, azathioprine, fludarabine, 5-fluorouracile, capecitabine, cytarabine, gemcitabine, preferably paclitaxel or gemcitabine.

In an embodiment X₃ is a miRNA coding proteins of therapeutic interest such as miRNA members of the miR-371-373 and miR-302/367 groups (Syring, I.; Bartels, J.; Holdenrieder, S.; Kristiansen, G.; Müller, S. C.; Ellinger, J. Circulating Serum MiRNA (MiR-367-3p, MiR-371a-3p, MiR-372-3p and MiR-373-3p) as Biomarkers in Patients with Testicular Germ Cell Cancer. Journal of Urology 2015, 193 (1), 331-337) which are highly expressed in all testicular cancers, regardless of patient age, tumour site or histological subtype. The quantification of circulating miRNAs has the potential to address unmet clinical needs in testicular tumours and provide major benefits to patients.

The bond between Y and A is either covalent or non-covalent, preferably the bond is covalent bond and more preferably an organometallic bond. The bonds between A and E, and E and X₃ are amide bond.

The possibility of combining different types of bioactive ligands by using a mixture of bioactive ligands on the same bioactivated metal nanocluster allows exploiting simultaneously the different functionalities of the different ligands. According to a specific embodiment, bioactive ligands are a mixture of bioactive ligands. For example, the mixture of bioactive ligands can be a mixture of markers and medicines. Specifically, in a therapeutic application in cancer, the bioactivated metal nanocluster can comprise matrix ligands, bioactive ligands which are markers and bioactive ligands which are medicines. Therefore the marker will be able to target a particular motif such as a receptor, and once the marker has recognized its target, the drug will be able to treat it. For example, the bioactive ligands can be either markers or medicines or mixtures thereof.

According to another embodiment, the bioactivated metal nanocluster has a proportion of the bioactive ligands of formula (Ia) at the surface of the bioactivated metal nanocluster of between 0,5% to 10%, preferably between 1% to 6%, more preferably between 3% to 5 %, the proportion of the bioactive ligands of formula (Ia) added to the proportion of the matrix ligands of formula (Ib) representing 100% at the surface of the bioactivated metal nanocluster. According to another embodiment, the proportion of the bioactive ligands of formula (Ic) at the surface of the bioactivated metal nanocluster is between 0,5% to 10%, preferably between 1% to 6%, more preferably between 3% to 5%, the proportion of the bioactive ligands of formula (Ic) added to the proportion of the matrix ligands of formula (Ib) representing 100% at the surface of the bioactivated metal nanocluster.

In another embodiment, the proportion of the bioactive ligands of formula (Ia) at the surface of the bioactivated metal nanocluster of between 0,5% to 10%, preferably between 1% to 6%, more preferably between 3% to 5 %, and the proportion of the bioactive ligands of formula (Ic) at the surface of the bioactivated metal nanocluster is between 0,5% to 10 %, preferably between 1% to 6%, more preferably between 3% to 5%, the proportion of the bioactive ligands of formula (Ia) and (Ic) added to the proportion of the matrix ligands of formula (Ib) representing 100% at the surface of the bioactivated metal nanocluster.

### Pharmaceutical composition comprising the bioactivated metal nanocluster of the invention

In another aspect, the invention relates to a pharmaceutical composition comprising:
a. The bioactivated metal nanoclusters of the invention, and
b. At least one pharmaceutical acceptable excipient.

In an embodiment, the bioactivated metal nanoclusters are in solid form. Preferably, the bioactivated metal nanoclusters are in thin particulate form. More preferably, the bioactivated nanoclusters are lyophilized to keep their integrity at about -20°C.

The at least one pharmaceutical acceptable excipient can be any of the excipients conventionally used in the pharmaceutical field such as surfactants, emulsifiers, lipids, hydrophilic or lipophilic gelling agents, hydrophilic or lipophilic additives, preservatives, antioxidants, solvents, bulking agent, chelating agents, colouring materials, and other pharmaceutical acceptable excipient.

Preferably the pharmaceutical composition comprises at least one excipient at content between 30% and 99%, preferably between 50% and 98% and more preferably between 90% and 97% and even more preferably about 95% by weight relative to the total amount of said composition.

In an embodiment the at least one pharmaceutical excipient is in liquid form. Therefore, the composition of the invention can be in the form of any dispersion comprising a solid phase such as a suspension such as a colloid, an aerosol, or foam.

In another embodiment, the bioactivated metal nanoclusters are in solid form and are encapsulated in a carrier system such as liposome or polymeric nanoparticle. Liposomes refer to vesicles consisting of a phospholipid bilayer on the periphery of an aqueous core, these liposomes generally serve to transport and protect active ingredients and are active ingredient delivery agent. Nanoparticles are either nanospheres that are "solid" spheres or nanocapsules that have a liquid or gelled core. These nanoparticles protect, isolate and allow a gradual release of the active ingredient they contain. Preferably, the bioactivated metal nanoclusters are encapsulated in any of the carrier system compatible with the hydrophilic properties of the bioactivated metal nanocluster. More preferably the bioactivated metal nanoclusters are encapsulated in multilamellar lipidic structures such as multilamellar vesicles (MLVs).

In an embodiment the at least one pharmaceutical excipient is in liquid form. Preferably the at least one pharmaceutical excipient is any excipient compatible with the carrier system chosen. Therefore, the composition of the invention can be in the form of any dispersion comprising a vehicular phase such as an emulsion or a suspension.

### Synthesis of the bioactivated metal nanocluster of the invention

The synthesis of the invention aims at producing in one step with not much reagent, a highly fluorescent bioactivated NCs. To do that, ligands of the invention have the capacity to be either a reducing agent, either stabilizing agent while being a matrix and/or bioactive ligands. Therefore the method of the invention does not involve any other reducing agent. Ligands of the invention will reduce de metal in order to form a compact shell in only one step.

It is another object of the present invention to provide a method for the synthesis of the bioactivated metal nanocluster of the invention, wherein said method comprises the following steps :
a. Providing, an aqueous solution comprising at least the ligands of formula (Ib) as defined in the invention and water,
b. Adding a solution of the metal salts selected from the group consisting of gold, copper, silver, platinum, and nickel, preferably a solution of gold salts of formula HAuCl₄,
c. Reacting during sufficient period of time at a temperature between 20°C and 200°C, preferably 70°C to 150°C, more preferably about 120°C,
d. Recovering the bioactivated metal nanocluster formed, and
e. Optionally, purifying the bioactivated metal nanocluster formed.

The step c of reacting during sufficient period of time at a temperature between 20°C and 200°C, preferably 70°C to 150°C, more preferably about 120°C allows to reduce the synthesis time and to tune the nanocluster size. As specified in articles Kawasaki, H.; Hamaguchi, K.; Osaka, I.; Arakawa, R. Ph-Dependent Synthesis of Pepsin-Mediated Gold Nanoclusters with Blue Green and Red Fluorescent Emission. Advanced Functional Materials 2011, 21 (18), 3508-3515 and Yu, Y.; Chen, X.; Yao, Q.; Yu, Y.; Yan, N.; Xie, J. Scalable and Precise Synthesis of Thiolated Au 10-12, Au 15, Au 18, and Au 25 Nanoclusters via PH Controlled CO Reduction. Chemistry of Materials 2013, 25 (6), 946-952*,* the pH is also a parameter that allows to tune the nanocluster size. Indeed, in the present invention, the more basic the pH, the smaller the size of nanoclusters. In an embodiment the step c of reacting is done with a pH of between 13 and 3, preferably between 5 and 4.

In another embodiment the sufficient period of time of step c. is between 1 minute to 24 hours, preferably between 1 hour to 12 hours, more preferably between 2 hours to 6 hours, even more preferably about 4 hours.

In an embodiment, the step a. of the method further comprises an aqueous solution of ligands of formula (Ia) and/or(Ic) as defined above.

In an embodiment, when the aqueous solution of ligands of formula (Ib) and (Ia) and/or(Ic) have an anchor A composed of (L) or (D) amino acid comprising a thiol function, and the solution of the metal is a solution of gold salts of formula HAuCl₄, these ligands have so much affinity to gold thanks to the S-Au bond, that the electron transfer between the ligands and the gold will increase the fluorescence emitted by the bioactivated metal nanocluster of the invention.

In an embodiment, the method further comprises step e. purifying the bioactivated metal nanocluster formed. Purifying can consist in method widely used in laboratory. For example, a method used in the laboratory to purify NCs consists in using filters combining centrifugation and size exclusion chromatography such as NAP-5 Sephadex^{™} G-25 columns purchased by Fisher Scientific, or filtration such as Amicon commercial purification system and particularly Amicon Ultra-2, Membrane Ultracel-3, PMNL 3 kDa purchased by Merck. The presence of very small pores in the filter allows ligands to be selectively released by centrifugation without allowing NCs to pass through.

In an embodiment, the filter has a cut-off threshold of 3kDa, such as the Amicon Ultracel 3kDa which has a pore size sufficient to purify red emitting NCs (diameter ≈ 17 nm) but too large to filter blue emitting NCs (diameter ≈ 1 nm). This makes it possible to separate the two types of NCs in a mixture, which is a very interesting point in terms of purification after synthesis.

In another embodiment, the filter has a cut-off threshold of 1kDa, such as Amicon Ultra-2, Membrane Ultracel-3, PMNL 1 kDa purchased by Merck. This makes it possible to purify the smaller blue emitting NCs. These ones with a size greater than 1kDa, will be retained by the filter. It is the retentate which will be recovered at the end of the purification.

In an embodiment, the step e of purifying the bioactivated metal nanocluster formed consists of a filtration with a cut-off threshold of 3kDa and/or a filtration with a cut-off threshold of 1kDa.

In another embodiment, the method does not comprise step e of purifying the bioactivated metal nanocluster. Indeed, excess ligand helps stabilize the NCs and increase their fluorescence. To preserve the NCs in the long term, a lyophilized suspension is an interesting formulation for industrial use or for certain analytical techniques.

In an embodiment, the method further comprises a freeze-drying step. This freeze-drying step can comprise the following steps:
- Freeze the bioactivated metal nanocluster thus recovered in liquid nitrogen,
- Place the bioactivated metal nanocluster frozen under high vacuum and pressure,
- Recover the solid particles of the bioactivated metal nanocluster thus freeze-dried,
- Optionally, suspend the bioactivated metal nanocluster thus freeze-dried and check under UV irradiation the presence or not of aggregate.

In an embodiment, the temperature of the freeze-drying step is between -70°C and -30°C, preferably between, -60°C and -40°C, more preferably between -55°C and -45°C, and even more preferably about -50°C.

In an embodiment, the pressure of the freeze-drying step is between 0.05mbar and 0.15 mbar, preferably between 0.075 mbar and 0.125 mbar, more preferably around 0.1 mbar.

In an embodiment, the time required for freeze-drying a suspension containing 3 mL of water is between 2 hours and 6 hours, preferably between 3 hours and 5 hours, more preferably around 4 hours.

The present invention further relates to a bioactivated metal nanocluster of the invention obtainable by the method of the invention, wherein it has a stability at 4°C at least 1 month, preferably at least 6 months, more preferably at least 12 months, even more preferably between 1 to 24 months, even more preferably between 4 to 18 months, even more preferably between 6 to 12 months.

The stability can be assessed by evaluating the capacity of the bioactivated metal nanocluster to disperse correctly in water. The test consists in redispersing the bioactivated metal nanocluster in water, performing a size-dependent filtration with a very low cut off of about 3kDa, and, observing under UV irradiation if the bioactivated metal nanocluster passes through the filter. If they are well dispersed, no fluorescence is lost before and after filtration. Another possible test consists in observing under UV irradiation the fluorescence of the bioactivated metal nanocluster before and after the filtration. The presence of aggregates indicates that the bioactivated metal nanocluster is not stable.

### Use of the bioactivated metal nanocluster of the invention or composition thereof

Another aspect of the invention relates to a bioactivated metal nanocluster of the invention or a composition of the invention for use as a medicament.

In another aspect, the invention relates to a bioactivated metal nanocluster of the invention or a composition of the invention for use in a method for treatment of a cancer in a subject in need thereof. Preferably the cancer is selected from the group comprising testicular cancer, and pancreatic cancer.

In another aspect, the invention relates to the use of a bioactivated metal nanocluster of the invention or a composition of the invention for the manufacture of a medicament for treating a cancer in a subject in need thereof.

In another aspect, the invention relates to a method for treating a cancer in a subject in need thereof, said method comprising the step of administering a bioactivated metal nanocluster of the invention or a composition of the invention to said subject in need thereof.

In another embodiment present invention further relates to a bioactivated metal nanocluster of the invention or a composition of the invention for use in a diagnostic method of a cancer in a subject in need thereof comprising the steps of administering a bioactivated metal nanocluster of the invention or a composition of the invention to said subject in need thereof, and acquiring an image of said subject.

The invention also relates to an *in vitro* diagnostic method of a cancer comprising acquiring an image of a subject sample comprising a bioactivated metal nanocluster of the invention or a composition of the invention.

In another aspect, the invention relates to the use of a bioactivated metal nanocluster of the invention or a composition of the invention for the manufacture of a medicament for detecting a cancer in a subject in need thereof.

In another aspect, the invention relates to a method for detecting a cancer in a subject in need thereof, said method comprising the step of: administering a bioactivated metal nanocluster of the invention or a composition of the invention to said subject in need thereof, acquiring an image of said subject.

Preferably, the diagnostic or detection is made by imaging. More preferably, optical fluorescence, X-ray, radiography, magnetic resonance imaging (MRI) and spectroscopy (MRS), single-photon-emission computed tomography (SPECT). Indeed, the imaging methods involving gold are optical fluorescence, X-ray, radiography, magnetic resonance imaging (MRI) and spectroscopy (MRS), single-photon-emission computed tomography (SPECT). When gold radioisotopes are used the imaging method will be the SPECT imaging.

Such bioactivated metal nanocluster may be ready for use as an injectable solution, preferably for in vivo detection of tumors by imaging in a subject in need thereof.

The requirements for effective pharmaceutical carriers for injectable compositions are well-known to those of ordinary skill in the art.

In certain aspects the subject is a mammal, for example but not limited to a rodent, canine, feline, or primate. In preferred aspects, the subject is a human.

In specific embodiments, an efficient amount of the bioactivated metal nanocluster for the diagnosis is administered to said subject 1 time per imaging.

Preferably, imaging may be acquired 1 to 70 hours, more preferably between 20 to 40 hours, after the intravenous administration of the bioactivated metal nanocluster to the subject, and even more preferably about 24 hours after the administration of the bioactivated metal nanocluster to the subject. The minimum recommended time to wait before starting imaging is 1 hour after the intravenous administration.

### LISTING OF FIGURES

**Figure 1****: Fluorescence Evolution of A-Cys3-E6-Asp** ; **Excitation 405 nm, during the synthesis at T=120°C; x=wavelength of the fluorescence emitted; y= fluorescence intensity emitted.**
**Figure 2****: Absorbance evolution of Au-Cys3-E6-Asp during the synthesis; T=120°C; x=wavelength of the fluorescence emitted; y= fluorescence intensity emitted.**
**Figure 3****: Fluorescence evolution of the mixture of Au-Cys3-E6-Asp and Au-Cys3-E6-U₁₁; Excitation : 405 nm; x=wavelength of the fluorescence emitted; y= fluorescence intensity emitted.**
**Figure 4****: Absorbance evolution of the mixture of Au-Cys3-E6-Asp and Au-Cys3-E6-U₁₁; Excitation : 405 nm; x=wavelength of the fluorescence emitted; y= fluorescence intensity emitted.**
**Figure 5** **: HR-TEM Images of gold NCs with 97% of matrix ligand Cys3-E6-Asp and 3% of Cys3-E6-U₁₁ ligand.**
**Figure 6** **: Size Distribution of gold NCs with 97% of matrix ligand Cys3-E6-Asp and 3% of Cys3-E6-U₁₁ ligand.**
**Figure 7** **: Cytometry tumor pancreatic cells with A. Cells + blue NCs (0%U11); B. Cells + blue NCs (1%U11); C. Cells + blue NCs (3%U11); x= fluorescence intensity; y= the number of cells.**
**Figure 8****: Fluorescence optical microscope image of pancreatic cancer cells (PANC1) incubated in the presence of (a) NCs of example 1 (0%U11) (b) NCs of example 2 (3% U11).**
**Figure 9****: Fluorescence optical microscope image of pancreatic cell nuclei (PANC1) labeled with NCs of example 2 (3% U11).**
**Figure 10****: HR-TEM Images of GSH NCs.**
**Figure 11****: Size Distribution of GSH NCs.**
**Figure 12****: Cytometry tumor pancreatic cells with Cells + GSHNCs; x= fluorescence intensity; y= the number of cells.**
**Figure 13****: Microscope images of early embryo stage zebra fish eggs after six hours of incubation in the presence of GSH NCs, Au-Cys3-E6-Asp NCs or without NCs (control)**
**Figure 14****: Microscope images of early embryo stage zebra fish embryo after an hour of incubation in presence of Au-Cys3-E6-Asp NCs with different excitation wavelength: a, (a) blue fluorescence showing the presence of fluorescent white spots, (b) red fluorescence showing no fluorescence as expected from these NC, (c) phase contrast images showing the corresponding embryo without any excitation for fluorescence**
**Figure 15****: Microscope images of GUV encapsulation with A. GUV (DOPC)+ Au-Cys3-E6-Asp NCs and B. GUV (DOPC/DOTAP)+ Au-Cys3-E6-Asp NCs.**
**Figure 16****: Microscope images of GUV encapsulation with GUV (DOPC)+ GSH NCs.**

### EXAMPLES

Hereinafter, the present disclosure is described in more details.

### Example 1: Synthesis of the fluorescent metallic nanocluster linked to matrix ligands

The matrix ligand used in this example is Aspartic acid. The capacity of the ligand to be a reducing agent as well as a ligand has been tested to achieve a one-step synthesis without the use of further reducing agent and while having good fluorescence.

### Synthesis of Au-Cys₃-E₆-Asp

Reactants are Cys₃-E₆-Asp of formula A and HAuCl₄.

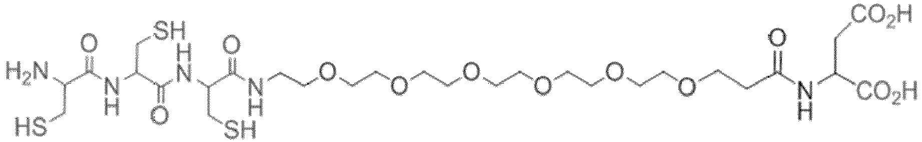

### Chemical Structure of Formula A: Cys₃-E₆-Asp

The Cys₃-E₆-Asp of formula A is prepared according the article *"*Quantum Dots to Target Polyhistidine-Tagged Proteins" J. AM. CHEM. SOC., vol.131, no. 41, 2009;14738-14746. Gold salts HAuCl₄ are purchased from Sigma Aldrich.

A freshly prepared aqueous solution of Cys₃-E₆-Asp of formula A (20 mM, 0.585 mL) were mixed with 2.115 mL of ultrapure water. The solution was heated in oil bath at 120°C and HAuCl₄ (20 mM, 0.3 mL) and was rapidly added to it. The reaction was stopped after stirring for 4h. An aqueous solution of the blue-emitting bioactivated nanoclusters (NCs) Au-CyS₃-E₆-Asp was formed. 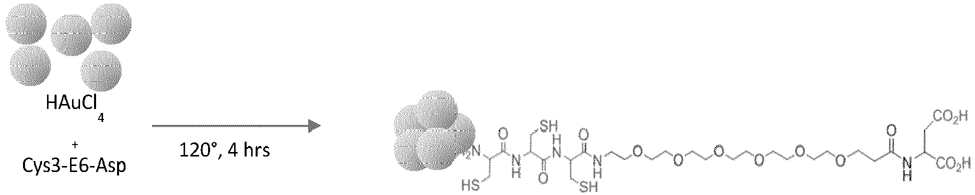 *This representation shows only the binding of a single ligand to a gold particle for hindrance concerns.*

### Fluorescence test using a spectrofluorimeter

To be used in imagery, the gold NCs have to possess good fluorescence properties.

The gold NCs solution thus formed have been tested trough a spectrofluorimeter in order to evaluate their fluorescence properties. This technique allows to evaluate the fluorescence of NCs.

### Protocol

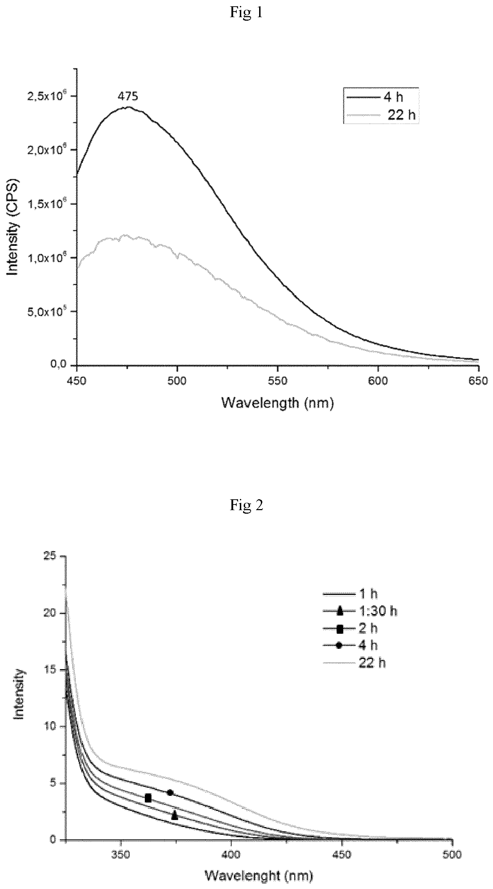

The gold NCs solution obtained after the synthesis is poured inside the cuvette of the spectrofluorimeter without being diluted. The excitation wavelength used is 405 nm and slits size of 2 nm. Results are shown in Figure 1.

### Results

Figure 1 shows that fluorescence of gold NCs is high after 4 hours of synthesis. These spectra demonstrate the generation of blue emitting (475 nm peak) gold NCs in the presence of the Cys₃-E₆-Asp ligand alone is possible quickly and without the use of further reactant such as, a reducing agent. However, if the reaction is allowed to continue for long periods of time, such as 22 hours, the gold NCs degrade and consequently the fluorescence as well.

The temporal evolution of absorbance during the reaction was also studied. Results are shown in Figure 2. Figure 2 demonstrates that the absorbance continues to increase even after long reaction times showing that the quantum efficiency decreases considerably, as the sample absorbs more photons but emits fewer fluorescence photons.

This test demonstrates that it is possible to obtain in a one-step synthesis of 4 hours without using any other reducing agent, a gold NCs with a fluorescence high enough to be used in imaging.

### Example 2: Synthesis of the fluorescent metallic nanocluster linked to a mixture of matrix ligands and bioactive ligands

In order to be able to use the gold NCs for targeting, it is necessary to functionalize the surface of the NCs via recognition segments such as a bioactive ligand.

### 1. U₁₁ as bioactive ligand

An important point was to verify that the addition of a bioactive ligand recognized by cancer cells did not significantly affect the fluorescence properties of the NCs of example 1, so that they could be used as markers for imaging.

The bioactive ligand used in this example is U₁₁ and the matrix ligand is aspartic acid. The U11 peptide is known for its ability to recognize uPAR overexpressed in pancreatic cancer cells. The synthesis conducted for this purpose uses Cys₃-E₆-Asp of formula A and 1% and 3% of U11 peptide. Different percentages have been tested in order to optimize the ratio between the two ligands for the best fluorescence signal.

When U₁₁ is used at a proportion of 3%, the resulted proportion of each ligand at the surface of the gold NCs is 3% for the receptor ligand expressed by cancer cells with U11 and 97% for the matrix ligand with Aspartic acid.

### One-step synthesis of Au-Cys₃-E₆-Asp/U₁₁

Reactants are Cys3-E6-Asp of formula A, HAuC14 and U11 peptide of formula B.

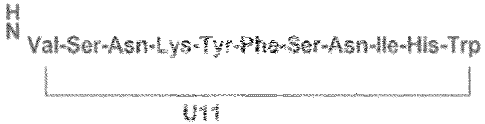

### Chemical Structure of formula B: U11

The Cys₃-E₆-Asp of formula A is prepared according the article *"*Quantum Dots to Target Polyhistidine-Tagged Proteins" J. AM. CHEM. SOC., vol.131, no. 41, 2009;14738-14746. Gold salts HAuCl₄ and U11 peptide are purchased respectively from Sigma Aldrich and Agentide Inc (USA).

### Au-Cys₃-E₆- Asp/U₁₁ 1 mol%

A freshly prepared aqueous solution of Cys₃-E₆-Asp of formula A (1.98 eq, 20 mM, 0.595 mL, 11,9 µmol) were mixed with a solution of U11 (0.02 eq, 1 mM, 0.12 mL, 0.12 µmol) and 1.485 mL of ultrapure water. The solution was heated in oil bath at 120°C and HAuC14 (1 eq, 20 mM, 0.3 mL, 6 µmol) and was rapidly added to it. The reaction was stopped after stirring for 4h. An aqueous solution of blue-emitting Au-Cys₃-E₆-U₁₁ 1 mol% NCs was formed.

### Au-Cys₃-E₆- Asp/U₁₁ 3 mol%

A freshly prepared aqueous solution of Cys₃-E₆-Asp of formula A (1.95 eq, 20 mM, 0.585 mL, 11.7 µmol) were mixed with a solution of U11 (0.06 eq,1 mM, 0.35 mL, 0.35 µmol) and 1.765 mL of ultrapure water. The solution was heated in oil bath at 120°C and HAuC14 (1 eq, 20 mM, 0.3 mL, 6 µmol) and was rapidly added to it. The reaction was stopped after stirring for 4h. An aqueous solution of blue-emitting Au-Cys₃-E₆-U₁₁ 3 mol% NCs was formed. 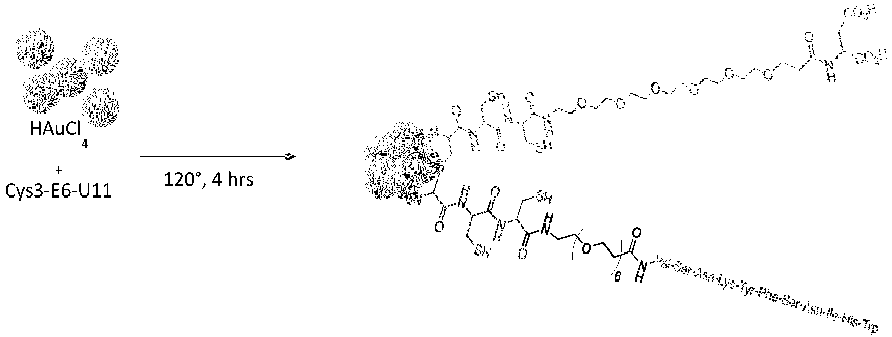 *This representation shows only the binding of a single bioactive ligand to gold a particle and a single matrix ligand to a gold particle for hindrance concerns.*

### Fluorescence test using a spectrofluorimeter

The same protocol of example 1 has been used in this example with two samples containing 1% and 3% mol of U11 previously obtained.

### Results

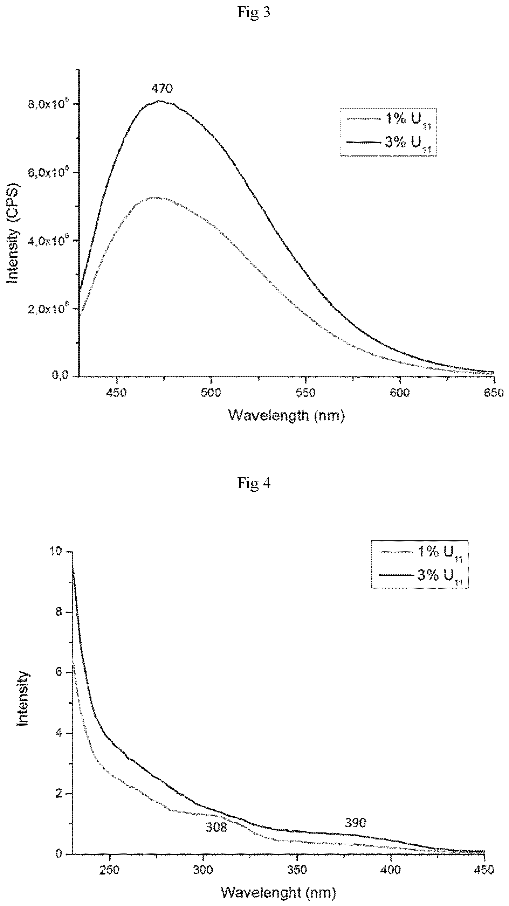

Figure 3 and Figure 4 show respectively the fluorescence and absorbance of NCs synthesized with 1% and 3% mol of U11. As in example 1, gold NCs have a strong fluorescence in the blue region after synthesis. However, the comparison of figures 1 and 3 demonstrates that the addition of U11 in the one-step synthesis generates an increase in blue-emitting (470nm peak) fluorescence for all percentages tested. Particularly, the fluorescence intensity has doubled with 1% of U11 and quadrupled with 3% of U11 compared to the gold NCs of example 1. The highest fluorescence intensity occurs with 3% U11 which also has an absorbance greater than the excitation wavelength (405 nm).

This test demonstrates that the addition of a bioactive ligand does not significantly affect the fluorescence properties of the gold NCs. Further, the addition of U11 peptide enhances the fluorescence. It is therefore possible to obtain in a one-step synthesis of 4 hours without using any other reducing agent, bioactivated gold NCs with a fluorescence high enough to be used in imaging.

### 2. Calcium channel blocker as bioactive ligand

Another important point was to verify that the addition of a bioactive ligand that recognizes calcium channel such as ORAI1 did not significantly affect the fluorescence properties of the NCs of example 1, so that they could be used as markers to diagnose cancer.

The bioactive ligand used in this example is the compound 1-[4-(4-Methoxyphenethoxy)phenethyl]-1H-benzo[d]imidazole of formula A (called A hereafter):

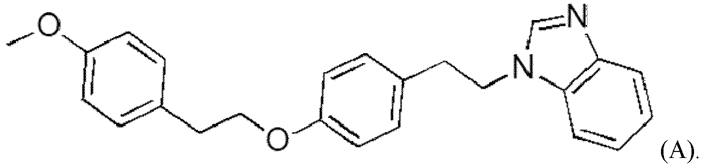

The 1-[4-(4-Methoxyphenethoxy)phenethyl]-1H-benzo[d]imidazole compound is obtained through the following synthesis : to a vigorous stirred (550 rpm) solution of benzimidazole (2 mmol) in 3.4 ml of DMF was added portion-wise sodium hydride 60% dispersion in mineral oil (0.081 g, 2 mmol) and the resulting suspension is stirred during 5 minutes. After addition of 4-(4-methoxyphenethoxy)phenethyl methanesulfonate (1 mmol) in one portion, the reaction mixture is heated at 60°C during 12 hours. After cooling down to room temperature, deionised water (34 ml) was added to the reaction mixture, the flask was shaken manually and then stored at 4°C (refrigerator) during 6 hours until complete precipitation. The insoluble material was recovered by filtration in a Buchner funnel (porosity N°4) and the precipitate was washed successively with deionised water (3 x 17 ml) and hexane (3 x 7 ml). The resulting solid was further dried under high vacuum (10⁻³ Torr) and afforded 0.307 g of the desired compound of formula A as white powder in 77% yield. Mp = 116-118°C.

In order to graft the bioactive ligand onto the spacer, which in this case is composed of 6 ethylene glycol units, the bioactive ligand as described above is substituted on its ether side with NH₂.

### Example 3: Study of the size of the gold NCs from example 2 using HR-TEM

The sample with the highest luminescence emission (3%) of the example 2 was analysed with HR-TEM technique to study the size of the gold NCs. High-resolution transmission electron microscopy (HR-TEM) is an imaging modality of transmission electron microscopes (TEM) that allows direct imaging of the atomic structure of the sample. The sample was imaged with a TEM (JEM 1400 JEOL, 120 kV) equipped with an ORIUS 1000 camera from the University of Rennes 1 (TEM platform Mric-UMS 3480-Biosit-University of Rennes1). The images were taken in HR-TEM mode in order to allow the imaging of objects of the order of a few nanometres and to check their crystallinity.

### Sample preparation

The sample was deposited and left to dry on appropriate copper grids according to the following procedure:
- Place a sheet of aluminium foil covered with a parafilm;
- Place a drop of 40 µl of the sample on the parafilm;
- Place a grid on the drop, taking care to bring the functional part of the grid in contact with the sample and wait 3 minutes;
- Remove the grid with tweezers and absorb the excess product with a piece of paper before putting it in the case;
- Let the sample to dry for about 24 hours before looking at it under the microscope HR-TEM.

### Results

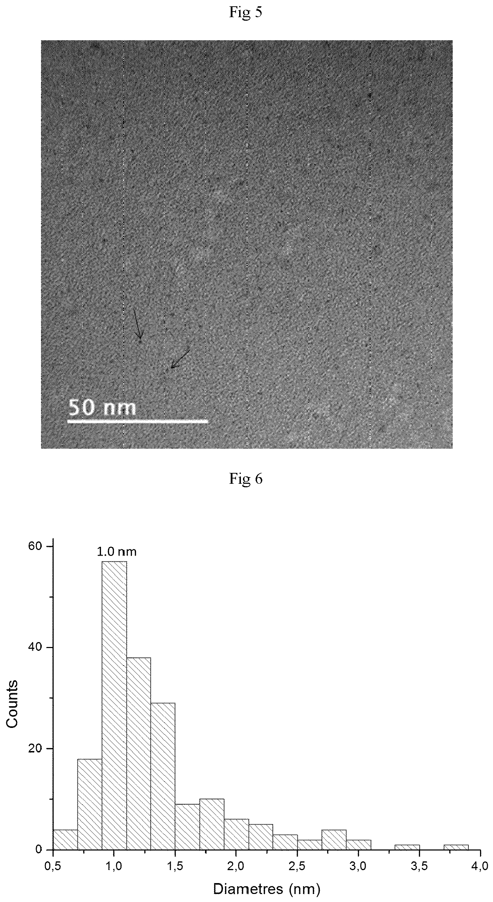

Figure 5 shows that the gold NCs with 3% of U11 are so small that they are barely distinguishable. In fact, as shown in Figure 6, they have an average size of 1 nm and the peak is quite thin, indicating that the dimensions of the gold NCs are rather uniform in the sample. This example demonstrates that the presence of U11 peptide, with its 3 thiol groups, creates small bioactivated gold NCs that emit in the blue.

Therefore this example demonstrates that the synthesis of the invention allows generating bioactivated gold NCs of very small size, in a single step, while obtaining sufficient fluorescence to be used in imaging.

The gold NCs of the invention have the capacity to be used as markers for imaging and could be used, for example, as novel markers for the diagnosis of cancer diseases.

### Example 4: Study of targeting efficiency of the gold NCs from example 2

In addition to the influence of the different percentages of U11 on fluorescence of the gold NCs, an extremely important point to study is the percentage allowing a better targeting efficiency.

It has been assessed the capacity of gold NCs of examples 1 and 2 (with and without U11 peptide) to recognize and position themselves on the wall of pancreatic tumor cells. To assess this capacity, cytometry and fluorescence optical microscope techniques have been used.

### 1. Cytometry

### Sample preparation:

A solution containing 2 billion pancreatic cells is centrifuged at 2000 rpm for 7 minutes, after which the supernatant is removed. 1.2 ml of calcium-containing buffer is added to the centrifuged cells. 200 µL of the resulting solution is added to each well, as well as 100 µL of the gold NCs solution, Au-Cys3-E6-Asp NCs, (at a concentration in the µM range).

The 2 billion cells in the solution are left to incubate with the gold NCs solution for 30 minutes in the "Non-Tissue Culture" wells. At the end of the 30 minutes, washing is carried out by adding 4 ml of buffer to well containing the incubated sample, centrifuging at 2000 rpm for 7 minutes and removing the supernatant.

The same protocol has been used in this example for three samples of gold NC of example 2 containing 0%, 1% and 3% of U11.

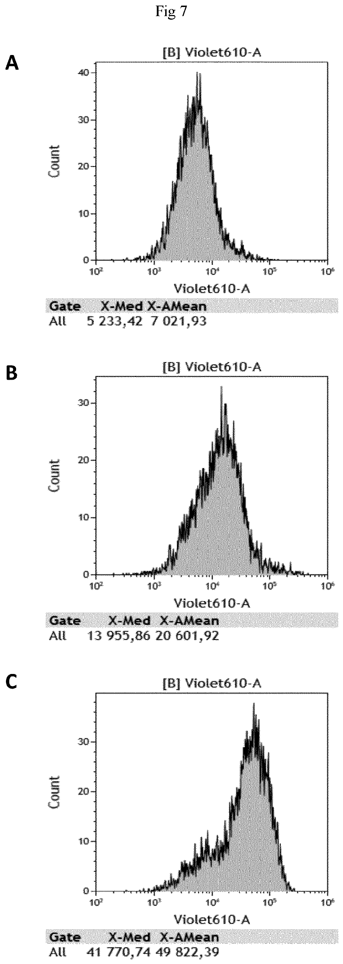

In the figure 7, the results of the measurement for Cys3-E6-Asp NCs (NCs with 0% of ligand U11), Cys3-E6-Asp(99%)/U11(1%) NCs (NCs with 1% of ligand U11), and Cys3-E6-Asp(97%)/U11(3%) NCs (NCs with 3% of ligand U11) are presented. The red and green graphs represent two different cell populations.

Figure 7 demonstrates, as in example 2, that the addition of U11 at the surface of gold NCs shifts the peak at higher fluorescence intensities (right shift of the peak in graph B compared to graph A).

In addition, in Figure C, the right shift continues to increase indicating that the number of cells with higher fluorescence intensity increases when the percentage of U11 increases. This demonstrates that, at higher percentages of Ull, the gold NCs adhere more to the cell wall and increase its fluorescence.

In conclusion, the 3% U11 gold NCs are the optimal percentage allowing a better targeting efficiency.

### 2. Fluorescence optical microscope

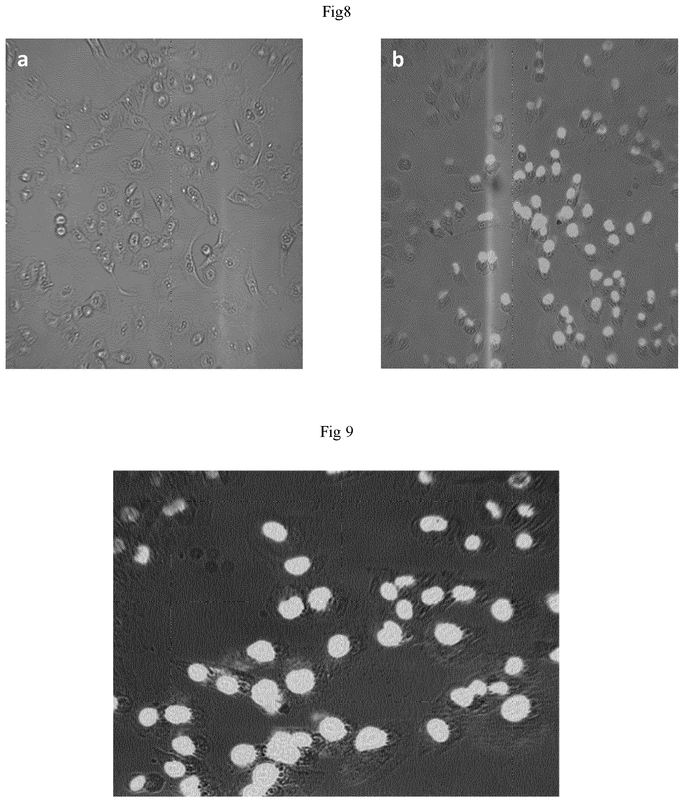

Image of samples with 0% and 3% U11 were obtained in Figure 8 (a) and (b) respectively. As can be learned from Fig. 8, under the same conditions and at the same concentrations, only the NCs containing U11 are able to mark the cancer cells demonstrating that U11 is a good marker for pancreatic cancer cells. By zooming the image b of figure 8 and looking at it in more detail in Figure 9, it is demonstrated that the NCs of example 2 with 3% U11 do not simply position themselves on the membrane, but pass membranes until it reaches the inside of the nucleus without killing cells. As demonstrated by example 3, it is thanks to the ultrasmall dimensions of about 1.5 nm of the gold NCs obtained through the synthesis of this invention, that they manage to overcome the cell membrane and position themselves inside the nucleus of the cell still alive.

This result is extremely important since it is now possible with the gold NCs of the invention to target protein presents in the nuclei while maintaining the cells targeted alive. Therefore a perspective to provide oligonucleotide, such as ARN, in the nucleus of targeted cells is possible.

Thanks to the one-step synthesis according to the invention, it is possible to obtain very small gold NCs capable of passing the membranes and targeting the nucleus. Therefore, the gold NCs of the invention are good candidates for the development of markers or drugs useful in the imaging and therapy of diseases such as cancer.

### Example 4: Comparative example with Au-GSH

The peptide currently most used for the synthesis of gold NCs is glutathione (GSH). This peptide is both ligand (via the SH group) and reducer. Glutathione is composed of three amino acids, including cysteine: this amino acid allows the ligand to be anchored to gold through the sulphide (SH) group, which is also capable of reducing gold and interacting with gold so as to anchor glutathione to gold by a strong Au-S bond.

The known and used protocol for the chemical synthesis of GSH AuNCs is described in the literature Luo, Z.; Yuan, X.; Yu, Y.; Zhang, Q.; Leong, D. T; Lee, J. Y.; Xie, J. From Aggregation-Induced Emission of Au(I)-Thiolate Complexes to Ultrabright Au(0)@Au(I)-Thiolate Core-Shell Nanoclusters. Journal of the American Chemical Society 2012, 134 (40), 16662-16670. https://doi.org/10.1021/ja306199p*.* However, this synthesis does not aim to functionalize gold NCs i.e. gold NCs capable of targeting via recognition segments, therefore further steps have to be done to render the gold NCs useful in imaging and therapeutic.

### Fluorescence test using cytometry technique

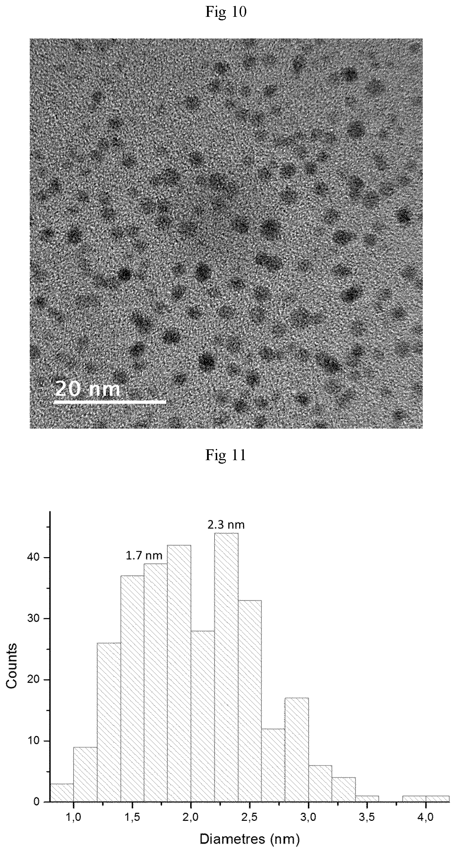

The same protocol of example 4.1 has been used in this example with a sample containing Au-GSH NCs prepared as described in the above mentioned literature. Their fluorescence properties are shown in Figure 10.

### Results

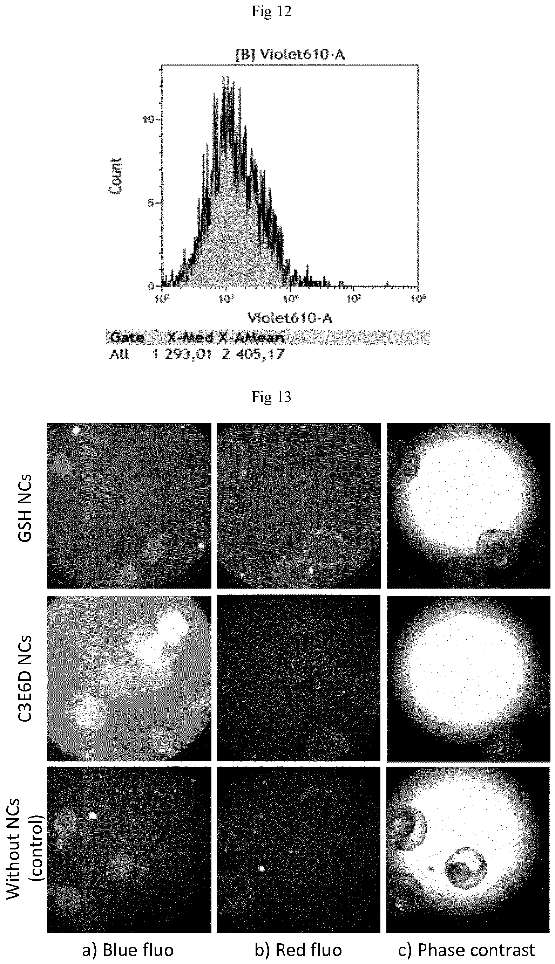

Figure 10 and 11 show that GSH NCs are heavier than the gold NCs of the invention (figures 5 and 6). Both Figure 12 and the control (Figure 7A) in which NCs are not present, show a similar spectrum, this indicates that the incubation of GSH NCs had no effect on the increase in the number of cells with higher fluorescence. These results can be explained by the fact that the GSH NCs are not functionalized; therefore their targeting properties are scarce, unlike the NCs of the invention.

### Example 5: Toxicity tests

Prior to any in vivo application of the NCs of example 2 with 3% U11, several toxicity tests must be conducted.

A toxicity test was performed using the eggs of a transparent fish called Zebrafish currently used in many toxicity studies. For our experiments we used the early embryo stage and added the NCs to the marine solution in which the Zebrafish live.

A first experiment consisted in studying the penetrability of NCs against the protective envelop formed by the egg wall (the Chorion).

A further experiment consisted in studying the penetrability of NCs in the embryo without its Chorion. In this experiment, the protective shell of the egg is no longer present.

### Protocol

All the experiments have been carried out on the Biosit plateform at the University Rennes 1 (SFR MS CNRS3480 -INSERM 018) with the technical assistance of Remy Le Guével.

### 1. Buffer preparation

The buffer used is a 135mM solution of NaCl, 5mM of KCl, 1 mM of MgCl, 10 mM of HEPES and 10 mM of glucose. The pH was brought to 7.4 by adding 3mL of 1M NaOH solution.

### 2. Incubation of PANC 1 cells with NCs

The wells containing pancreatic cancer cells (PANC 1) were washed twice using the culture medium. After emptying the wells, 50 µL of a NCs solution was added at a standard concentration C, that obtained following the synthesis. The cells are left to incubate together with the NCs solution for one hour at 37 ° C, isolating with parafilm. At the end of the incubation period two more washes are carried out using the buffer solution mentioned above. At the end of the washing, each well is filled with 200 µL of buffer solution and the cells are viewed under a fluorescence light microscope.

### 3. Incubation of zebrafish eggs with NCs

Initially, using an optical microscope, the eggs still alive were chosen, distinguishable from the inside of a yellow color. In each well, 3 eggs selected in this way were inserted together with 80 µL of marine solution and 80 µL of a standard concentration NCs solution (concentration C) to reach a final concentration of C/2. To test also a different concentration other wells were filled with 133 µL of marine solution and 27 µL of the solution of NCs at standard concentration C to obtain a final concentration of C/6. We immediately moved to the fluorescence light microscope to follow the evolution of egg growth and the influence of NCs. Eggs were observed daily for the next 3 days.

### 4. Incubation of Zebrafish embryos with NCs

In each well, 5 selected Zebrafish embryos already released from the chorion were inserted together with 187 µL of marine solution and 63 µL of a standard concentration NCs solution to reach a final concentration of C/4. To test also a different concentration other wells were filled with 208 µL of marine solution and 42 µL of the solution of NCs at standard concentration to reach a final concentration of C/6. We immediately underwent a fluorescence light microscope to follow the evolution of the influence of NCs on Zebrafish embryos. Embryos were observed daily for the next 3 days.

### Results

### • Penetrability of NCs in the zebra fish egg

Results are shown in figure 13. Through this experiment it has been noticed that NCs with red fluorescence (GSH NCs) cannot overcome the chorion barrier and position themselves on it. On the contrary, gold NCs of the invention with blue fluorescence (Au-Cys3-E6-Asp NCs) manage to overcome it and do not accumulate in any particular area, showing only a background of diffuse blue fluorescence. This difference is certainly attributable to the size of the NCs. Since as already known in the literature Braunbeck, T.; Kais, B.; Lammer, E.; Otte, J.; Schneider, K.; Stengel, D.; Strecker, R. The Fish Embryo Test (FET): Origin, Applications, and Future. Environmental Science and Pollution Research 2015, 22 (21), 16247-16261*,* the chorion allows only molecules smaller than 3kDa to pass, a condition respected by the blue particles but not by the red ones.

Different concentrations of NCs (C, C/2, C/4, C/6) were tested, and as expected, we had less toxicity for lower NCs concentrations.

### • Penetrability of NCs in zebra fish embryo (eggs without the chorion)

These embryos are not yet able to feed themselves and use the pouch positioned under the head to do so. In this experiment, the protective shell of the egg is no longer present; therefore the fish are more exposed to NCs, even to NCs with red luminescence that previously could not overcome the chorion.

Regardless of the type of NCs, greater toxicity was shown in all cases when incubating with non-purified NCs. It has been observed that he NCs with red fluorescence, GSH NCs, cannot penetrate inside the Zebrafish, and this result is the same regardless of the incubation time and the concentration of the NCs in the marine solution.

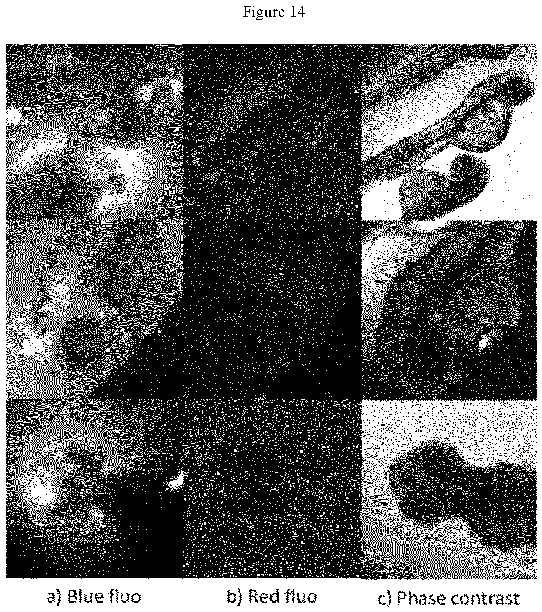

Carrying out the same experiment in the presence of blue fluorescent NCs of the invention (C3E6D NCs) the result is totally different. Indeed, as can be seen in figure 14, these smaller blue NCs, corresponding to white spots in the figure, are able to enter the fish in times that depend on the concentration of NCs used: for higher concentrations there are shorter internalization times, using the same type of NCs with the same ligand. In addition, it is noted that the blue fluorescence initially appears in the head and then passes into the rest of the body. This positioning could be due to the fact that the only way for NCs to enter at this fish growth stage through its gills. The mouth is not yet a way of access since at this stage nourishment still takes place through the bag.

Since the exposure time required for the internalisation of blue fluorescent NCs does not lead to excessive mortality, it should be possible to interrupt the exposure after the internalization has occurred and thus block the effects of toxicity due to too long exposure times.

Both the GSH NCs and the NCs of the invention, Au-Cys3-E6- Asp/U11 3 mol%, after an hour of incubation with the Zebrafish, give a low mortality. The most obvious difference between the two types of NCs is that in the case of GSH NCs there is no internalization, regardless of the concentration and incubation time, unlike the Au-Cys3-E6- Asp/U11 3 mol% of the invention which are already internalized after one hour of incubation.

### Example 6: formulation of the gold NCs of example 2

### 1. Lyophilisation

To preserve NCs in the long term, a lyophilized suspension of the gold NCs is an interesting formulation for industrial use or for certain analytical techniques.

### Protocol

The aqueous suspension of NCs is first frozen in liquid nitrogen, then the flask is placed under high vacuum under pressure and temperature conditions that allow the water contained in the sample to sublimate.

After 24 hours, it is checked that the fluorescence is preserved and especially that the particles can be resuspended in the water without any problem of aggregation at the desired concentration.

### 2. Encapsulation

A method of transporting gold NCs of the invention is to encapsulate them in GUVs (Giant Unilamellar Vesicles).

### Protocol

The GUV formation protocol follows a procedure described in the article *Matsushita-*Ishiodori, Y.; Hanczyc, M. M.; Wang, A.; Szostak, J. W.; Yomo, T. Using Imaging Flow Cytometry to Quantify and Optimize Giant Vesicle Production by Water-in-Oil Emulsion Transfer Methods. Langmuir 2019, 35 (6), 2375-2382*.*

### Samples preparations

Initially, 4 solutions are prepared:
1. Inner solution 25 µL of sucrose solution (1 M) + 25 µL of gold NCs solution of example 1 or GSH NCs of example 5;
2. Stock solution (glucose 500 mM);
3. Lipid solution.

The lipid solution (3) has been prepared using neutral lipids DOPC or DOPC + 5% DOTAP positive. In both cases, the lipids are dissolved in chloroform to give a final concentration of 10 mg/mL. Then 20 µL of this solution in chloroform is added to 1800 µL of paraffin oil in a 5 mL flask and the solution is heated at 80°C for 30 min without stopper. In this way, the chloroform evaporates and causes the creation of a lipid solution in the oil. To make sure that all the chloroform has evaporated, the solutions are placed in the desiccator for 20 min. The lipid solution (3) is ready.

### GUV preparation

50 µL of solution (1) + 400 µL of solution (3) are placed in an eppendorf and vortexed for 40s to form a water-in-oil (w/o) emulsion. In a second eppendorf, 200 µL of (2) is introduced, to which the newly formed emulsion is gently added.

Wait 10 minutes and place the emulsion for overnight incubation. The GUVs then formed on the bottom and are removed the next day to another Eppendorf and redispersed in 300 µL of solution (2). The GUVs are placed for overnight incubation. The next day, the bottom is taken up again and the GUVs are ready to be stored in the refrigerator.

### Results

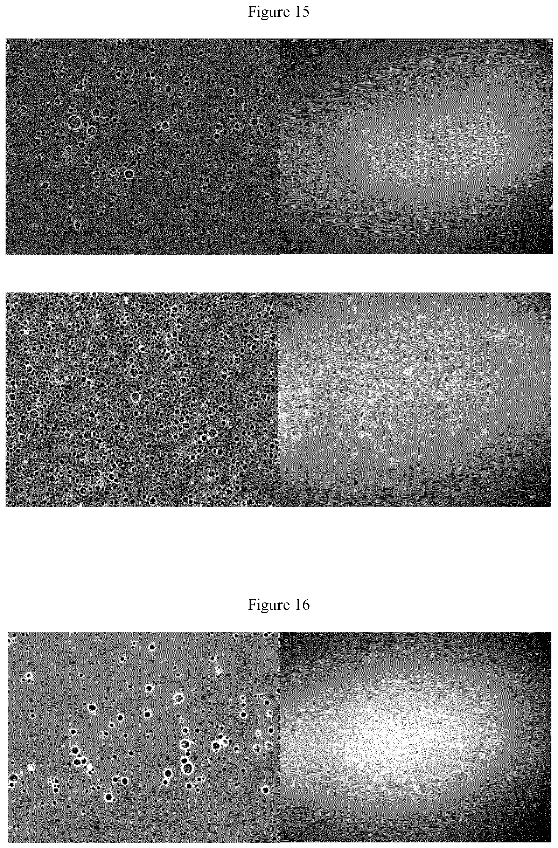

Results are shown in figures 14 and 15. Figure 14 shows strong fluorescence only inside the GUVs, where they are encapsulated. The GUVs are also stable and there is no formation of aggregates with the NCs of the invention. Figure 15 shows the formation of aggregates which, having a stronger fluorescence, masks that of the rest of the encapsulated GSH NCs.

This is due to the fact that GSH NCs are less stable as they tend to aggregate more easily than gold NCs of example 1. As gold NCs of example 1 have surface ligands with longer chains (thanks to PEGs), they have steric stabilization compared to GSH NCs. In addition they also have electrostatic stabilization. Therefore the aggregation of NCs of the invention is well more difficult. The best results are obtained with gold NCs of the invention Figure 14.

## Claims

1. A bioactivated metal nanocluster comprising a mixture of ligands linked to a fluorescent metallic nanocluster, wherein the mixture comprises at least ligands linked to a fluorescent metallic nanocluster of formula (Ib) :
Y-AₚE_{n'}(X₂) _{(Ib)}
wherein
Y, being on the N terminal side of A, is the fluorescent metallic nanocluster wherein the metal is selected from the group consisting of gold, copper, silver, platinum, and nickel;
AₚE_{n'}(X₂), is a matrix ligand of formula (Ib);
A is an anchor composed of an (L) or (D) amino acid selected from the group comprising Glycine (Gly), Alanine (Ala), Valine (Val), Cysteine (Cys), Proline (Pro), Leucine (Leu), Isoleucine (Ile), Methionine (Met), Tryptophan (Trp), Phenylalanine (Phe), Lysine (Lys), Arginine (Arg), Histidine (His), Threonine (Thr), Tyrosine (Tyr), Serine (Ser), Asparagin (Asn), Glutamine (Gln), Aspartic Acid (Asp), Glutamic acid (Glu), and mixture thereof;
p is an integer greater than 1, preferably between 1 to 10, more preferably between 2 and 6, even more preferably 3 ;
E, being on the C terminal side of A, is a spacer composed of ethylene glycol unit repeated n' times;
n' is an integer greater than or equal to 1, preferably between 2 to 20, more preferably between 3 and 8, even more preferably 4 or 6;and
X₂ is a matrix ligand selected from the group comprising at least one (L) or (D) amino acid or a primary amine (-RNH₂) or alcohol (-RCH₂OH) or alkyl carboxylic acid (-RCO₂H) or alkyl ether group (-ROR'), wherein the matrix ligand does not contain thiol function and R and R' are short alkyl chain.

2. The bioactivated metal nanocluster of claim 1, wherein it further comprises bioactive ligands of formula (Ia) or (Ic) or mixtures thereof:
Y-AₚEₙ(X₁) _{(Ia)}
Y-AₚE_{n"}(X₃) _{(Ic)}
wherein
Y, being on the N terminal side of A, is the fluorescent metallic nanocluster wherein the metal is selected from the group consisting of gold, copper, silver, platinum, and nickel;
AₚEₙ(X₁), is a bioactive ligand of formula (Ia);
AₚE_{n"}(X₃), is a bioactive ligand of formula (Ic);
A is an anchor composed of an (L) or (D) amino acid selected from the group comprising Glycine (Gly), Alanine (Ala), Valine (Val), Cysteine (Cys), Proline (Pro), Leucine (Leu), Isoleucine (Ile), Methionine (Met), Tryptophan (Trp), Phenylalanine (Phe), Lysine (Lys), Arginine (Arg), Histidine (His), Threonine (Thr), Tyrosine (Tyr), Serine (Ser), Asparagin (Asn), Glutamine (Gln), Aspartic Acid (Asp), Glutamic acid (Glu), and mixture thereof;
p is an integer greater than 1, preferably between 1 to 10, more preferably between 2 and 6, even more preferably 3 ;
E, being on the C terminal side of A, is a spacer composed of ethylene glycol unit repeated n or n" times;
n is an integer greater than or equal to 1, preferably between 2 to 20, more preferably between 3 and 8, even more preferably 4 or 6;
n" is an integer greater than or equal to 1, preferably between 2 to 20, more preferably between 3 and 8, even more preferably 4 or 6 ;
X₁ is a marker which is either a ligand interacting or interfering with ion channels selected from calcium channels, potassium channels, sodium channels, chlorine channels, either a receptor ligand expressed by cancer cells or exosomes selected from the group comprising at least one (L) or (D) amino acid, preferably U11 polypeptide, glycine-aspartate-arginine (RGD) or any sequence comprising RGD, or microRNAs identified as cancer markers, or antibodies addressed against the tetraspanin family proteins such as CD63, CD81 and CD9, wherein the marker does not contain thiol function; and
X₃ is a medicine selected from the group comprising anticancer medicines and ion channel blockers, an oligonucleotide selected from the group comprising anticancer medicines, or microRNAs coding proteins of therapeutic interest, wherein the medicine or the oligonucleotide does not contain thiol function.

3. The bioactivated metal nanocluster of claim 1 or 2, wherein it comprises mixture of ligands linked to a fluorescent metallic nanocluster of formula (Ia) and (Ib) or (Ia), (Ib) and (Ic) or (Ia) and (Ic).

4. The bioactivated metal nanocluster of claims 2 or 3, wherein the proportion of the bioactive ligands of formula (Ia) at the surface of the bioactivated metal nanocluster is between 0,5% to 10%, preferably between 1% to 6%, more preferably between 3% to 5%, the proportion of the bioactive ligands of formula (Ia) added to the proportion of the matrix ligands of formula (Ib) representing 100% at the surface of the bioactivated metal nanocluster.

5. The bioactivated metal nanocluster of claims 2 or 3, wherein the proportion of the bioactive ligands of formula (Ic) at the surface of the bioactivated metal nanocluster is between 0,5% to 10%, preferably between 1% to 6%, more preferably between 3% to 5%, the proportion of the bioactive ligands of formula (Ic) added to the proportion of the matrix ligands of formula (Ib) representing 100% at the surface of the bioactivated metal nanocluster.

6. The bioactivated metal nanocluster of claims 2 or 3, wherein the proportion of the bioactive ligands of formula (Ia) at the surface of the bioactivated metal nanocluster of between 0,5% to 10%, preferably between 1% to 6%, more preferably between 3% to 5 %, and the proportion of the bioactive ligands of formula (Ic) at the surface of the bioactivated metal nanocluster is between 0,5% to 10%, preferably between 1% to 6%, more preferably between 3% to 5%, the proportion of the bioactive ligands of formula (Ia) and (Ic) added to the proportion of the matrix ligands of formula (Ib) representing 100% at the surface of the bioactivated metal nanocluster.

7. A pharmaceutical composition comprising :
a. The bioactivated metal nanocluster as defined in claims 1 to 6,
b. At least one pharmaceutical acceptable excipient.

8. A method for the synthesis of the bioactivated metal nanocluster of claims 1 to 6, wherein said method comprises the following steps :
a. Providing, an aqueous solution comprising at least the ligands of formula (Ib) as defined in claim 1 and water,
b. Adding a solution of the metal salts selected from the group consisting of gold, copper, silver, platinum, and nickel, preferably a solution of gold salts of formula HAuCl₄,
c. Reacting during sufficient period of time at a temperature between 20°C and 200°C, preferably 70°C to 150°C, more preferably about 120°C,
d. Recovering the bioactivated metal nanocluster formed, and
e. Optionally, purifying the bioactivated metal nanocluster formed.

9. The method of claim 8 wherein the step a. further comprises an aqueous solution of ligands of formula (Ia) and/or (Ic).

10. The method of claim 8 or 9 wherein the sufficient period of time of step c. is between 1 minute to 24 hours, preferably between 1 to 12h, more preferably between 2 to 6h, even more preferably about 4h.

11. The method of claims 8 to 10, wherein the synthesis does not involve any other reducing agent.

12. A bioactivated metal nanocluster of claims 1 to 6 obtainable by the method of claim 8 to 11, wherein it has a stability at 4°C of at least 1 month, preferably at least 6 months, more preferably at least 12 months, even more preferably between 1 to 24 months, even more preferably between 4 to 18 months, even more preferably between 6 to 12 months.

13. A bioactivated metal nanocluster of claims 1 to 6 or a composition of claim 7 for use as a medicament.

14. A bioactivated metal nanocluster of claims 1 to 6 or a composition of claim 7 for use in a method for treatment of a cancer in a subject in need thereof.

15. A bioactivated metal nanocluster of claims 1 to 6 or a composition of claim 7 for use in a diagnostic method of a cancer in a subject in need thereof comprising the steps of administering a bioactivated metal nanocluster of claims 1 to 6 or a composition of claim 7 to said subject in need thereof, and acquiring an image of said subject.
